# EUROPEAN PATENT APPLICATION

(11) **EP 2 347 834 A2**
(43) Date of publication of application: **27.07.2011**
(21) Application number: 09814742.4
(22) Date of filing: 27.08.2009
(51) Int. Cl.: B09B 3/00

(54) **ORGANIC WASTE TREATMENT SYSTEM**

(30) Priority: 22.09.2008 KR 20080092903
(71) Applicant: Park, Choong Kil, Seoul 135-091 (KR); Noguchi, Keizaburo, Saitama 350-0053 (JP)
(72) Inventor: Park, Choong Kil, Seoul 135-091 (KR); Noguchi, Keizaburo, Saitama 350-0053 (JP)
(74) Representative: Mammel, Ulrike
(86) International application number: PCT/KR2009/004772
(87) International publication number: WO 2010/032926

(57) **Abstract**

The present invention relates to an organic waste treatment apparatus for treating organic wastes with microbes. More specifically, this invention relates to a n organic waste treatment apparatus including a treatment tank having multiple treatment chambers, and a transfer unit for transferring the organic waste so that the organic waste inserted into the treatment tank is transferred through said multiple treatment chambers before being discharged from the treatment tank. Different types of microbes can be contained in separate treatment chambers, and thus different growth conditions for each type of microbe can be easily met.

## Description

### Technical Field

The present invention relates to an organic waste treatment apparatus which uses microbes.

### Background Art

Organic waste has up until the present mainly been treated by means of burying and incineration. However, in the case of burying, due to methane gas, leachate, etc., offensive odor s are generated from landfills and there is water or soil pollution. Incineration is problematic in that various pernicious ingredients, such as dioxin and so on, are discharged, bringing about secondary environmental pollution. Therefore, recently, a method of biologically decomposing organic waste with microbes and stabilizing it to make by-products which can be used as compost has been gained in popularity. Development and research into a treatment apparatus for embodying such a method have been active and made progress.

An organic waste treatment apparatus using microbes includes a treatment tank, a heating unit, an agitation unit, etc. Organic waste and microbes are input into a treatment chamber defined in the treatment tank. The organic waste input into the treatment chamber is heated, agitated and fermented by the heating unit, the agitation unit and the microbes. Providing an ideal fermentation environment is very important to making such a treatment processes smooth. The term "providing the ideal fermentation environment" refers to controlling growth conditions including factors, such as oxygen, water, humidity, temperature, etc., that activate microbes. If the growth conditions are properly maintained, the microbes required for fermentation can become active and proliferate. Thus, the microbes actively decompose organic waste and kill pathogenic bacteria which have been contained in the waste. After the fermentation process has been ran its course, the well-matured organic waste (that is, a by-product) becomes stable, and pathogenic bacteria die out. Therefore, the organic waste can be turned into clean compost which does not generate an offensive odor nor decay excessively rapidly.

However, the conventional organic waste treatment apparatus using microbes typically treats organic waste in a single space (in other words, a single treatment chamber). Thus, it is difficult to provide the distinct optimum growth conditions in response to the kind of microbes, d espite the fact that the treatment efficiency can be enhanced when several kinds of microbes are used. Therefore, there is a problem in that the treatment effect is not satisfactory.

### Disclosure

### Technical Problem

Accordingly, the present invention has been made keeping in mind the above problems occurring in the prior art, and an object of the present invention is to provide a n organic waste treatment apparatus which can easily create the ideal fermentation conditions.

### Technical Solution

In order to accomplish the above object, in an aspect, the present invention provides a n organic waste treatment apparatus, including: a treatment tank body providing a space for treating organic waste therein, with an inlet port and an outlet port respectively formed on opposite ends of the treatment tank body; at least one partition wall partitioning the space in the treatment tank body into a plurality of treatment chambers so that different kinds of microbes grow in different independent spaces, the partition wall being placed between the inlet port and the outlet port such that the treatment chambers are arranged in a line in a longitudinal direction of the treatment tank body, with a transfer hole formed th rough the partition wall so that the organic waste is transferred between the treatment chambers through the transfer hole; a rotating shaft provided in the treatment tank body in the longitudinal direction through the partition wall; a plurality of agitation and transfer blades provided on the rotating shaft in such a way that at least one thereof is disposed in each of the treatment chambers, so that when the rotating shaft rotates, the agitation and transfer blades agitate the organic waste input into th e treatment tank body and transfer the organic waste in an axial direction; and a shaft drive means for rotating the rotating shaft.

The organic waste treatment apparatus may further include an adhered waste detaching means for detaching organic waste that has adhered to the partition wall. Preferably, the adhered waste detaching means may include detaching blades provided on the rotating shaft in such a way that at least one thereof is disposed at each of both sides of the partition wall. Furthermore, ea ch of the detaching blades may include: a blade member provided up on the rotating shaft such that at least a portion of a front side of the blade member faces the partition wall; and a reinforcing member provided on a rear side of the blade member, the reinforcing member having a rib structure.

The organic waste treatment apparatus may further include an adhered waste detaching means for detaching organic waste that has adhered to inner surfaces of opposite end walls of the treatment tank body.

The treatment tank body may have a semicircular cross -sectional bottom which is convex downwards and is coaxial with the rotating shaft. Furthermore, the treatment tank body may have at least one inspection hole on an upper portion thereof. The inspection hole may be closed and opened by an inspection hole cover. The inspection hole cover may be configured in such a way that at least a portion thereof is transparent.

The partition wall may comprise three partition walls in such a way that the space in the treatment tank body is partitioned into four treatment chambers. The four treatment chambers may include: a first treatment chamber receiving organic waste input into the space through the inlet port; a fourth treatment chamber com municating with the outlet port; and second and third treatment chambers successively disposed between the first treatment chamber and the fourth treatment chamber.

The first partition wall disposed between the first treatment chamber and the second treatment chamber may be lower than the remaining partition walls. Furthermore, the third partition wall disposed between the third treatment chamber and the fourth treatment chamber may have a predetermined height so that an upper end thereof comes into contact with a ceiling of the treatment tank body. The fourth treatment chamber may comprise a maturing chamber in which the organic waste that has been fermented through the first through third treatment chambers is matured.

The transfer hole may be located above the rotating shaft within a turning radius of the agitation and transfer blades.

The agitation and transfer blade may be provided on the rotating shaft such that it is slant ed to the left and the right by a predetermined angle with respect to the axial direction. Each agitation and t ransfer blade may have a distal end which is wider in width. For example, the width-extended end structure of the agitation and transfer blade may assume a T shape. A reinforcing member having a rib structure may be provided on at least one of the two sides of each agitation and transfer blade.

The organic waste treatment apparatus may further include at least one selected from the group including a heating unit applying heat to the treatment tank body, and a deodorization unit connected to the treatment tank body.

In another aspect, the present invention provides an organic waste treatment apparatus, including: a treatment tank having a plurality of treatment chambers to grow different kinds of microbes in different independent spaces, an inlet port thro ugh which organic waste is input into one of the treatment chambers, and an outlet port through which the organic waste is discharged from another one of the treatment chambers; and an agitation and transfer unit agitating the organic waste input into the treatment tank and transferring the organic waste so that the organic waste passes through the treatment chambers before being discharged from the treatment tank.

### Advantageous Effects

The present invention can more effectively treat organic waste.

### Description of Drawings

Fig. 1 is a front view showing an organic waste treatment apparatus, according to the present invention ;
Fig. 2 is a left side view of Fig. 1;
Fig. 3 is a sectional view taken along line A -A of Fig. 2;
Fig. 4 is a sectional view taken alo ng line B-B of Fig. 2;
Figs. 5, 6 and 7 are perspective views showing partition walls of the organic waste treatment apparatus according to the present invention;
Fig. 8 is a perspective view showing a rotating shaft of the organic waste treatment apparatus, and agitation and transfer blades provided on the rotating shaft according to the present invention; and
Fig. 9 is an enlarged view of an agitation and transfer blade of Fig. 8.

### <Description of the elements in the drawings >

3: treatment tank 5: heating unit
7: deodorization unit 10: treatment tank body
11: inlet port 12: outlet port
21, 23, 25: partition wall 22, 24, 26: transfer hole
30: rotating shaft 40: agitation and transfer blades
50: shaft drive means 61, 65: detaching blade
S1∼S4: treatment chamber

### Best Mode

Hereinafter, a preferred embodiment of the present invention will be described in detail with reference to the attached drawings.

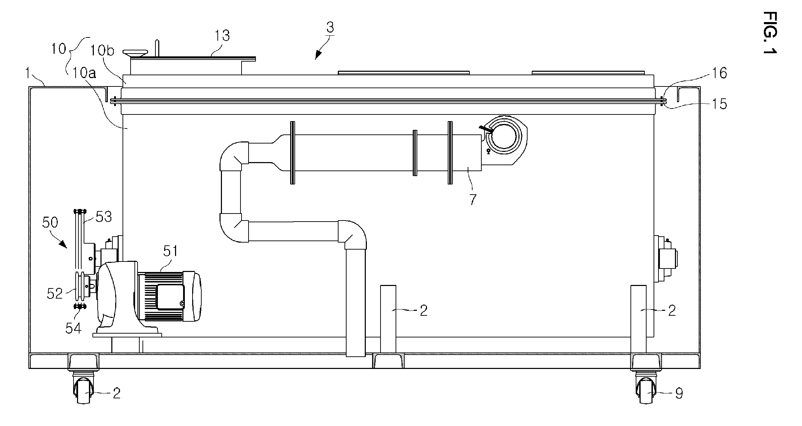

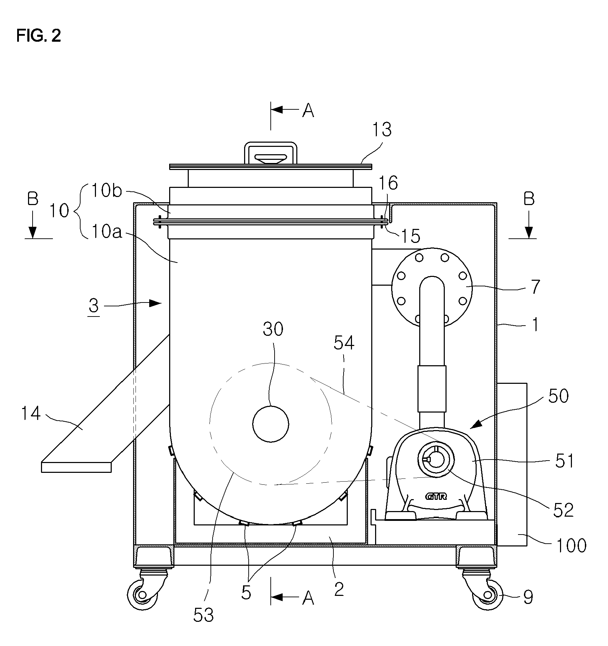

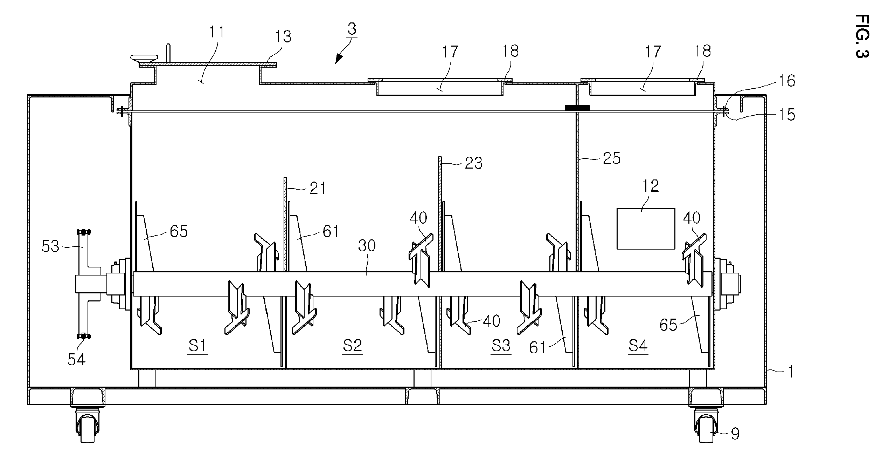

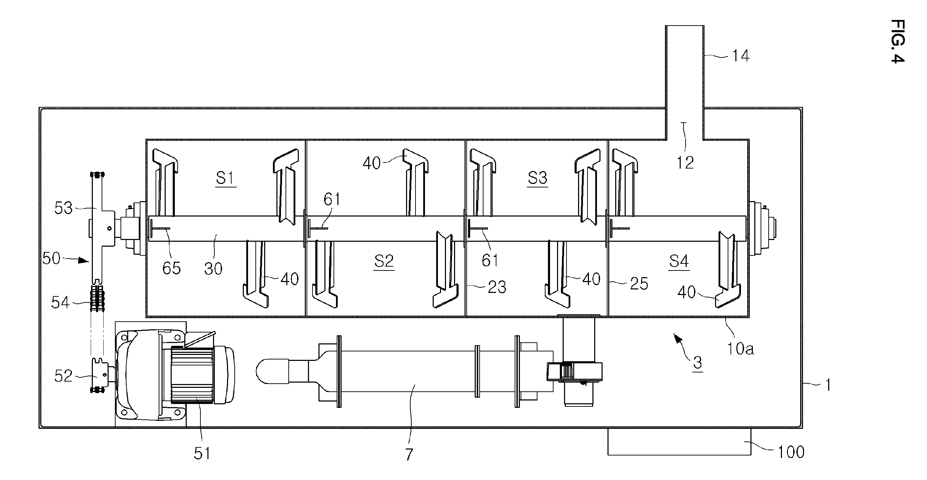

Fig. 1 is a front view showing an organic waste treatment apparatus, according to the present invention. Fig. 2 is a left side view of Fig. 1. Figs. 3 and 4 are sectional views taken along line A -A and line B-B of Fig. 2, respectively.

The organic waste treatment apparatus according to the present invention treats organic waste with microbes. Although the organic waste which can be treated by the organic waste treatment apparatus is not limited to a special kind of organic waste so long as it can produce a by - product that can be used as compost or the like after being processed by fermentation, the major targets include the food waste which is generated by residences, food-related companies or enterprises and excretions which are generated from livestock raising facilities.

As shown in Figs. 1 through 4, the organic waste treatment apparatus according to the present invention includes a treatment tank 3, an agitation and transfer unit, an adhered waste detaching means, a heating unit 5 and a deodorization unit 7. The treatment tank 3 has an internal space for treating organic waste therein. The agitation and transfer unit agitates organic waste input into the treatment tank 3 and, simultaneously, transfers the organic waste between an input side and an output side in a reciprocating manner. The adhered waste detaching means detaches, from an inner surface of the treatment tank 3, organic waste that has adhered to the inner surface of the treatment tank 3 while being agitated and transferred by the agitation and transfer unit . The heating unit 5 applies heat to the treatment tank 3. The deodorization unit 7 removes offensive odors generated when treating the organic waste.

The treatment tank 3 includes a treatment tank body 10 having the internal space in which organic waste is treated by microbes, and at least one or more partition walls 21, 23 and 25 which partit ion the internal space of the treatment tank body 10 into a plurality of treatment chambers S1 through S4. As such, in the present invention, the plurality of treatment chambers S1 through S4 are provided so that different kinds of microbes which are used to treat the organic waste are supplied into the respective treatment chambers S1 through S4. Thus, the different kinds of microbes can respectively act in independent spaces so that the different kinds of microbes can be prevented from interfering with each other. Moreover, if each of the treatment chambers S1 through S4 is configured such that conditions for growing the corresponding kind of microbes are independently controlled, the optimal growth conditions can be created in each treatment chamber S1, S2, S3, S4 for each corresponding kind of microbe so that the microbes can be grown under their optimal environment. Of course, this provision of the plurality of treatment chambers S1 through S4 can easily embody creating the optimal growth conditions for each kind of microbe.

The treatment tank body 10 includes a tank part 10a and a tank cover 10b. The tank part 10a has a rectangular shape which is longish in the horizontal direction in a plan view. In addition, the tank part 10a is open on an upper end thereof and has a bottom having a semicircular cross-section which is convex downwards. The tank cover 10b covers the open upper end of the tank part 10a. Furthermore, the treatment tank body 10 has, on both sides thereof, an inlet port 11 into which organic waste to be treated is input, and an outlet port 12 through which the treated organic waste is discharged from the tank body 10. In detail, the inlet port 11 is formed in one end of the tank cover 10b and is open ed and closed by an inlet port cover 13. The outlet port 12 is formed in a sidewall of the tank part 10a. An outlet pipe 14 is connected to the outlet port 12. An opening control means for opening or closing a passage of the outlet pipe 14 is provided on the outlet pipe 14 , although this is not shown in drawings.

The tank part 10a and the tank cover 10b respectively include flanges 15 and 16 which correspond to each other when the open upper end of the tank part 10a is covered by the tank cover 10b. The two flanges 15 and 16 are coupled to each other by fastening means, such as bolts, nuts, etc. Although not shown in the drawings, a sealing member, such as a packing, is interposed between the contact surfaces between the tank part 10a and the tank cover 10b or between the two flanges 15 and 16.

The partition walls 21, 23 and 25 are placed upright on the bottom of the tank part 10a so that the treatment chambers S1 through S4 are arranged in a line along the longitudinal direction of the tank part 10a. The partition walls 21, 23 and 25 a re located between the inlet port 11 and the outlet port 12. In this embodiment, the three partition walls 21, 23 and 25 are provided so that the internal space of the tank part 10a is partitioned into the four treatment chambers S1 through S4, in detail, as follows:
1. a first treatment chamber S1 is provided beneath the inlet port 11 so that organic waste input into the inlet port 11 is supplied into the first treatment chamber S1;
2. a fourth treatment chamber S4 is communicating with the outlet port 12; and
3. a second treatment chamber S2 and a third treatment chamber S3 are successively disposed between the first treatment chamber S1 and the fourth treatment chamber S4.

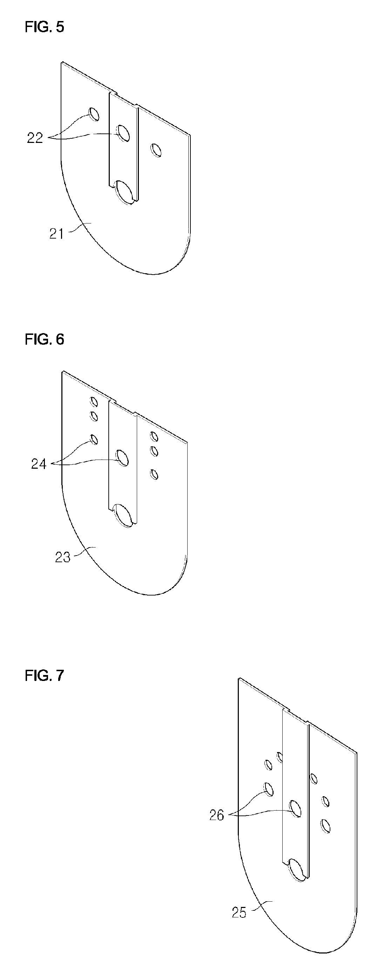

Figs. 5 through 7 respectively illustrate the partition walls 21, 23 and 25. As shown in these drawings, at least one transfer hole 22, 24, 26 is formed through each partition wall 21, 23, 25. The organic waste supplied into the first treatment chamber S1 is successively transferred via the second through fourth treatment chambers S2 through S4 to the outlet port 12 by the transferring operation of the agitation and transfer unit. Here, the organic waste is transferred to a subsequent treatment chamber through the corresponding transfer hole 22, 24, 26. Although the transfer holes 22, 24 and 26 of the partition walls 21, 23 and 25 are illustrated as being circular in Figs. 5 through 7, each transfer hole 22, 24, 26 may be various in shape, for example, a polygonal shape, an elliptical shape, etc. Furthermore, the disposition, the arrangement, the size, etc. of the transfer holes 22, 24 and 26 may also be changed in a variety of manners, as conditions require.

The first partition wall 21 which is disposed between the first treatment chamber S1 and the second treatment chamber S2 is low er than the second partition wall 23 and than the third partition wall 25. Due to this, organic waste can be prevented from being excessively supplied into the first treatment chamber S1. In other words, if an excessive amount of organic waste is input into the first treatment chamber S1, some of the organic waste flows over the first partition wall 21 from the first treatment chamber S1 into the second treatment chamber S2. Thereby, the amount with which organic waste is input into the first treatment chamber S1 can be controlled.

Of the treatment chambers S1 through S4 of the present invention, the first through third treatment chambers S1 through S3 are used as fermentation chambers, and the fourth treatment chamber S4 is used as a maturing chamber where the organic waste that has been fermented in the first through third treatment chambers S1 through S3 is matured.

The third partition wall 25 which is disposed between the third treatment chamber S3 and the fourth treatment chamber S4 has a predetermined height so that an upper end of the third partition wall 25 comes into contact with the tank cover 10b which forms the ceiling of the treatment tank body 10. In other words, the fourth treatment chamber S4 which is used as the maturing chamber is isolated from the remaining treatment chambers S1 through S3 such that the fourth treatment chamber S4 communicates with the remaining treatment chambers S1 through S3 only through the transfer hole 26 of the third partition wall 25. Thereby, the maturing operation in the fourth treatment chamber S4 can be more reliably carried out.

Various kinds of microbes can be used to treat organic waste. In this embodiment, filamentous fungi are used in the first treatment chamber S1, actinomycetes are used in the second treatment chamber S2, and bacilli are used in the third treatment chamber S3. That is, this embodiment is configured in such a way that different kinds of microbes respectively act in the first through third treatment chambers S1 through S3. For reference, during the operation of treating organic waste, filamentous fungi decompose glucoses, amino acids, etc., actinomycetes decompose celluloses, hemicelluloses, etc., and bacilli decompose fibers or the like.

Meanwhile, the treatment tank body 10 has at leas t one inspection hole 17. In this embodiment two inspection holes 17 are provided. The inspection holes 17 are formed in the tank cover 10b. One of the two inspection holes 17 is located above the second partition wall 23 provided between the first partition wall 21 and the third partition wall 25 (that is, between the second treatment chamber S2 and the third treatment chamber S3). The other inspection hole 17 is located above the fourth treatment chamber S4. Each inspection hole 17 is openably closed by an inspection hole cover 18. Preferably, the inspection hole cover 18 is configured in such a way that at least a portion thereof is made transparent using a transparent window or the like so that a user can observe the interior of the treatment tank body 10 through the transparent portion.

The agitation and transfer unit includes a rotating shaft 30, a plurality of agitation and transfer blades 40 and a shaft drive means 50. The rotating shaft 30 is provided in the treatment tank body 10 in the longitudinal direction through all the partition walls 21, 23 and 25. The agitation and transfer blades 40 are provided on the rotating shaft 30 in such a way that at least one is disposed in each treatment chamber S1, S2, S3, S4. The shaft drive means 50 rot ates the rotating shaft 30 in a forward or reverse direction.

The rotating shaft 30 is disposed such that the center thereof is consistent with (coaxial with) a center axis of the semicircular portion that forms the bottom of the tank part 10a. The rotating shaft 30 has an appropriate length so that opposite ends thereof protrude outside the tank part 10a through the opposite end walls of the tank part 10a. The opposite ends of the rotating shaft 30 are rotatably supported by bearings or the like.

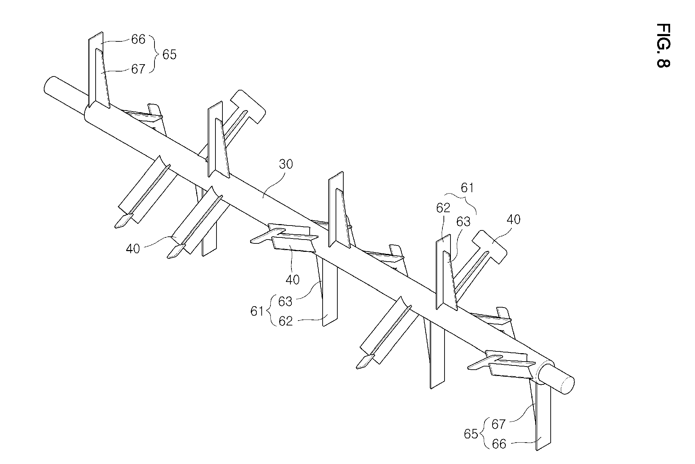

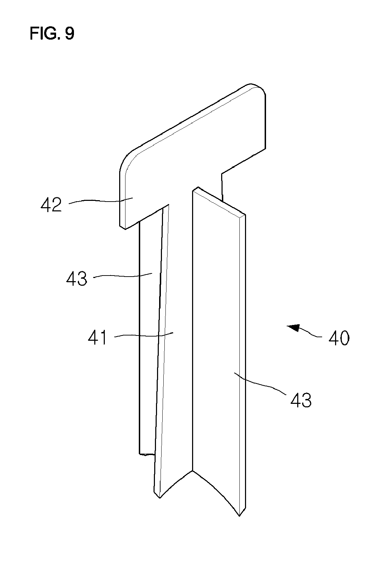

Fig. 8 is a perspective view showing the rotating shaft 30 and the agitation and transfer blades 40 provided on the rotating shaft 30. Fig. 9 is an enlarged perspective view of the agitation and transfer blade 40.

The agitation and transfer blades 40 function to agitate organic waste input into the treatment tank body 10 and to transfer the organic waste in the axial direction, that is, in the longitudinal direction of the treatment tank body 10. Each agitation and transfer blade 40 has a blade member which forms a structure capable of embodying the above functions. As shown in Figs. 8 and 9, the blade member of the agitation and transfer blade 40 is perpendicularly provided on the rotating shaft 30 and includes a vertical plate 41 which slants to the left and the right with respect to the axial direction at a predetermined angle, and a horizontal plate 42 which is integrally connected at a medial portion thereof to a distal end of the vertical plate 41 and extends to both sides of the vertical plate 41 in the widthwise direction to form a perpendicular structure. This structure imparts the agitation and transfer blade 40 with an overall T shape in which the width of the distal end thereof is extended , so that organic waste can be effectively agitated and transferred by the agitation and transfer blade 40. Furthermore, the turning radius of each agitation and transfer blade 40 is slightly less than a radius of the semicircular portion that forms the bottom of the tank part 10a. A planar reinforcing member 43 which serves as a rib is perpendicularly provided on at least one of the two side surfaces of the vertical plate 41. In this embodiment, two reinforcing members 43 are provided on the respective side surfaces of the vertical plate 41 in such a way that the reinforcing members 43 along with the vertical plate 41 form a criss-cross structure.

When the rotating shaft 30 is rotated by the operation of the shaft drive means 50, the agitation and transfer blades 40 rotate along with the rotating shaft 30. Organic waste input into the treatment tank body 10 is drawn up and agitated by the blade member and the reinforcing members 43 of the agitation and transfer blades 40 that are rotating ; the organic waste is simultaneously transferred in the axial direction. The amount of organic waste that each agitation and transfer blade 40 draws up can be increased by the horizontal plate 42 and the reinforcing members 43.

Meanwhile, the transfer holes 22, 24 and 26 of the partition wa Ils 21, 23 and 25 are located within the turning radius of the agitation and transfer blades 40 so that organic waste drawn up by the agitation and transfer blades 40 passes through the transfer holes 22, 24 and 26. Preferably, the transfer holes 22, 24 and 26 are located higher than the rotating shaft 30. Furthermore, in the same manner as the transfer holes 22, 24 and 26, the outlet port 12 of the tank part 10a is also located at a position at which organic waste drawn up by the agitation and transfer blades 40 can enter the outlet port 12.

The shaft drive means 50 includes a motor 51 and a power transmission unit which transmits rotating force from the motor 51 to the rotating shaft 30. The motor 51 has a motor shaft which is parallel to the rotating shaft 30. The power transmission unit incl udes a drive sprocket wheel 52 which is mounted to the motor shaft, a driven sprocket wheel 53 which is provided on the rotating shaft 30 and located outside the treatment tank 3, and a chain 54 wound around the drive sprocket wheel 52 and around the driven sprocket wheel 53. As such, the power transmission unit comprises a chain drive type power transmission unit. Of course, the power transmission unit may comprise a gear drive or belt drive type power transmission unit in place of the chain drive type.

Organic waste is generally adhesive. Therefore, during the agitation and transfer operation, organic waste may adhere to the opposite end walls of the tank part 10a or the partition wall 21, 23 or 25. The adhered waste detaching means which has been ment ioned above functions to detach the adhered organic waste from the wall. The adhered waste detaching means includes a first detaching means which detaches organic waste that has adhered to the partition walls 21, 23 and 25, and a second detaching means which detaches organic waste that has adhered to the opposite end walls of the tank part 10a.

The first detaching means includes detaching blades 61 which are provided on the rotating shaft 30 in such a way that at least one thereof is disposed close to each of both sides of the partition walls 21, 23 and 25. Each detaching blade 61 includes a planar blade member 62 which is perpendicularly provided on the rotating shaft 30 and faces the corresponding partition wall 21, 23, 25, and a planar reinforcing member 63 which is perpendicularly provided on a rear surface of the blade member 62 (a front surface of which faces the partition wall) and functions as a reinforcing rib (refer to Fig. 8). In the drawing, although the entirety of the front surface of the bla de member 62 is illustrated as closely facing the corresponding partition wall 21, 23, 25, the blade member 62 may be configured such that only a portion of the front surface thereof closely faces the partition wall 21, 23, 25. When the rotating shaft 30 rotates, the detaching blades 61 of the first detaching means rotate along with the rotating shaft 30. Organic waste that has adhered to the partition walls 21, 23 and 25 is detached therefrom by the blade members 62 of the detaching blades 61 which strike the organic waste. The detached organic waste fall down and then is agitated by the agitation and transfer blades 40. The reinforcing members 62 of the detaching blades 61 function to draw up and agitate the organic waste while the organic waste that has adhered to the partition walls 21, 23 and 25 is being detached therefrom.

Thanks to the detaching blades 61 having the above -mentioned structure, all of the organic waste can be involved in the agitation of the organic waste by the agitation and transfer blades 40 without omission. Furthermore, the efficiency of agitating organic waste can be further improved by the combined agitation operation of the agitation and transfer blades 40 and the reinforcing members 63. In addition, the structural strength of each detaching blade 61 can be enhanced by the presence of the reinforcing member 63. Hence, the detaching blades 61 can more reliably detach organic waste from the partition walls 21, 23 and 25 and agitate the organic waste. Further, each blade member 62 has a planar shape, and the reinforcing member 63 is perpendicularly provided on the rear surface of the blade member 62 , thereby facilitating maintaining the detaching blade 61 close to the corresponding partition wall 21, 23, 25 .

Meanwhile, the first detaching means may further include at least one of a vibrator which vibrates each partition wall 21, 23, 25, and an air nozzle which jets air onto both sides of each partition wall 21, 23, 25, although these are not illustrated in the drawings. Of course, the first detaching means may include only at least one of the vibrator and the air nozzle without including the detaching blade 61.

In the same manner as the first detaching means described above, the second detaching means includes a plurality of det aching blades 65. The detaching blades 65 of the second detaching means are mounted to the rotating shaft 30 in such a way that at least one thereof is disposed on the inner surface of each end wall of the tank part 10a. Furthermore, each detaching blade 65 of the second detaching means also includes a blade member 66 and a reinforcing member 67. Compared to the first detaching means, the blade member 66 and the reinforcing member 67 of the second detaching means have the same structure, and the operation and effect are those of the first detaching means, except for the fact that the blade member 66 and the reinforcing member 67 pertain to the end walls of the tank part 10a. Therefore, a detailed explanation will be omitted.

Furthermore, the second detac hing means may also further include at least one of a vibrator and an air nozzle in the same manner as the first detaching means. In addition, the second detaching means may include only at least one of the vibrator and the air nozzle without including th e detaching blade 65.

The heating unit 5 keeps the treatment chambers S1 through S4 at a predetermined temperature. The heating unit 5 includes at least one heater. For example, the heater may comprise four heaters. In this case, the heaters are provided on the bottom of the tank part 10a in such a way that one heater is disposed in a lower portion of each treatment chamber S1, S2, S3, S4. Of course, this case is configured such that the temperatures of the treatment chambers S1 through S4 are independe ntly controlled.

The deodorization unit 7 has a suction fan (not shown) and a filter (not shown) therein and is configured such that an end of a suction pipe of the deodorization unit 7 enters the third treatment chamber S3 through the sidewall of the third treatment chamber S3. In the deodorization unit 7 having this construction, when the suction fan is operated, air is sucked from the treatment chambers S1 through S4 into the deodorization unit 7 by the suction force of the suction fa n. The air sucked into the deodorization unit 7 is processed by the filter so that odor is removed from the air before it is discharged from the deodorization unit 7. Of course, during this process, vapor along with air is discharged from the treatment chambers S1 through S4. In other words, the deodorization unit 7 can also function to control the humidity in the treatment chambers S1 through S4. The filter is not limited to a special type filter, so long as it can effectively remove odors from air. In this embodiment, a platinum catalytic filter is used.

Meanwhile, unlike the construction described above, the deodorization unit 7 may include a plurality of suction pipes which are respectively connected to the treatment chambers S1 through S4.

In the drawings, reference numeral 1 denotes a casing. The casing 1 contains therein the elements described above, that is, the treatment tank 3, the agitation and transfer unit, the adhered waste detaching means, the heating unit 5 and the deodorization unit 7. The casing 1 has a structure which is open on the upper end thereof so that the tank cover 10b of the treatment tank 3 is exposed to the outside. As necessary, in place of the casing 1, a frame structure may be used.

Furthermore, reference numeral 9 denotes a caster. In t his embodiment, the caster 9 comprises a plurality of casters 9 which are mounted to the lower surface of the bottom of the casing 1 to provide mobility to the casing 1.

Reference numeral 100 denotes a control box. The organic waste treatment apparatus according to the present invention can be electronically controlled by the control box 100 so that organic waste input into the treatment tank body 10 is automatically treated. Automation using the control box 100 is useful in the following case.

If the motor 51 of the shaft drive means 50 is continuously operated only in one direction, some organic waste may be discharged from the organic waste treatment apparatus despite it not having been completely treated. On the other hand, if the motor 51 has not been operated for a long time, microbes cannot be activated due to a shortage of oxygen resulting from insufficient agitation. To avoid these problems and effectively treat organic waste, it is preferable that the motor 51 be set such that it is repeatedly operated in a sequence of forward rotation, stop and reverse rotation at predetermined time intervals (for example, intervals of 6 minutes).

Meanwhile, the treatment tank 3 is supported by a plurality of supports 2 so that it is spaced apart from the bottom of the casing 1 by a predetermined distance. The deodorization unit 7 includes a discharge pipe which has an end that extends to the outside through the bottom of the casing 1.

Although the preferred embodiment of the present invention has been disclosed for illustrative purposes, those skilled in the art will appreciate that various modifications, additions and substitutions are possible, without departing from the scope and spirit of the invention as disclosed in the accompanying claims.

For example, in the above embodiment, although the treatment chambers S1 through S4 have been illustrated as being arranged in a line, the treatment chambers S1 through S4 may be arranged in a line but in a shape bent at least once. Of course, in this case, the rotating shaft 30 must comprise a plurality of rotating shafts proportion al in number to the number of bends in the line along which the treatment chambers S1 through S4 are arranged. In addition, a power transmission means, such as gears or the like, must be pro vided between the rotating shafts to transmit rotating force therebetween.

Furthermore, if the rotating shaft 30 is excessively long, it may be divided into several parts to prevent it from sagging. In this case, a shaft coupling is interposed each between adjacent divided parts of the rotating shaft.

In addition, although the forgoing embodiment has been illustrated as having the single shaft drive means 50 and the rotating force of the shaft drive means 50 has been illustrated as being transmitted to one end of the rotating shaft 30, the organic waste treatment apparatus may include two shaft drive means 50 and be configured such that the rotating force of each shaft drive means 50 is transmitted to the corresponding one of both ends of the rotating shaft 30.

## Claims

1. An organic waste treatment apparatus, comprising:
a treatment tank body providing a space for treating organic waste therein, with an inlet port and an outlet port respectively formed on opposite ends of the treatment tank body;
at least one partition wall partitioning the space in the treatment tank body into a plurality of treatment chambers so that different kinds of microbes grow in different independent spaces, the partition wall being placed between the inlet port and the outlet port such that the treatment chambers are arranged in a line in a longitudinal direction of the treatment tank body, with a transfer hole form ed through the partition wall so that the organic waste is transferred between the treatment chambers through the transfer hole;
a rotating shaft provided in the treatment tank body in the longitudinal direction through the partition wall;
a plurality of agitation and transfer blades provided on the rotating shaft in such a way that at least one thereof is disposed in each of the treatment chambers, so that when the rotating shaft rotates, the agitation and transfer blades agitate the organic waste input into the treatment tank body and transfer the organic waste in an axial direction; and
shaft drive means for rotating the rotating shaft.

2. The organic waste treatment apparatus as set forth in claim 1, further comprising:
adhered waste detaching means for detaching organic waste that has adhered to the partition wall.

3. The organic waste treatment apparatus as set forth in claim 2, wherein the adhered waste detaching means comprises:
detaching blades provided on the rotating shaft in such a way that at least one thereof is disposed at each of both sides of the partition wall.

4. The organic waste treatment apparatus as set forth in claim 3, wherein each of the detaching blades comprises:
a blade member provided up on the rotating shaft such that at least a portion of a front side of the blade member faces the partition wall; and
a reinforcing member provided on a rear si de of the blade member, the reinforcing member having a rib structure.

5. The organic waste treatment apparatus as set forth in claim 1, further comprising:
adhered waste detaching means for detaching organic waste that has adhered to inner surfaces of op posite end walls of the treatment tank body.

6. The organic waste treatment apparatus as set forth in claim 1, wherein the partition wall comprises three partition walls in such a way that the space in the treatment tank body is partitioned into a first treatment chamber receiving organic waste input into the space through the inlet port, a fourth treatment chamber communicating with the outlet port, and second and third treatment chambers successively disposed between the first treatment chamber and the fourth treatment chamber.

7. The organic waste treatment apparatus as set forth in claim 6, wherein the first partition wall disposed between the first treatment chamber and the second treatment chamber is lower than the remaining partition walls.

8. The organic waste treatment apparatus as set forth in claim 6 or 7, wherein the third partition wall disposed between the third treatment chamber and the fourth treatment chamber has a predetermined height so that an upper end thereof comes into contact with a ceiling of the treatment tank body, and
the fourth treatment chamber comprises a maturing chamber in which the organic waste that has been fermented through the first through third treatment chambers is matured.

9. The organic waste treatment apparatus as set forth in claim 1, wherein the transfer hole is located above the rotating shaft within a turning radius of the agitation and transfer blades.

10. The organic waste treatment apparatus as set forth in claim 1, wherein each of the agitation and transf er blades has a distal end that is wider in width.

11. The organic waste treatment apparatus as set forth in claim 10, wherein a reinforcing member is provided on at least one of both sides of each of the agitation and transfer blades, the reinforcing mem ber having a rib structure.

12. The organic waste treatment apparatus as set forth in claim 1, further comprising:
a heating unit applying heat to the treatment tank body; and
a deodorization unit connected to the treatment tank body.

13. An organic waste treatment apparatus, comprising:
a treatment tank having a plurality of treatment chambers to grow different kinds of microbes in different independent spaces , an inlet port through which organic waste is input into one of the treatment chambers, and an outlet port through which the organic waste is discharged from another one of the treatment chambers; and
an agitation and transfer unit agitating the organic waste input into the treatment tank and transferring the organic waste so that the organic waste passes through the treatment chambers before being discharged from the treatment tank.
